Europäisches Patentamt

European Patent Office

Office européen des brevets

(11) Publication number: **0 363 019**
**A2**

## EUROPEAN PATENT APPLICATION

(21) Application number: **89309123.1**

(22) Date of filing: **08.09.89**

(51) Int. Cl.5: **A61F 2/36 , A61F 2/46**

(30) Priority: **09.09.88 GB 8821126**

(43) Date of publication of application:
**11.04.90 Bulletin 90/15**

(84) Designated Contracting States:
**AT BE CH DE ES FR GB GR IT LI LU NL SE**

(71) Applicant: **BIOMET LIMITED**
**Waterton Industrial Estate**
**Bridgend South Glamorgan CF31 3YN(GB)**

(72) Inventor: **Iversen, Björn Franc**
**Gammel Strandvey 94**
**3050 Humlebaek(DK)**

(74) Representative: **Jones, Michael Raymond et al**
**HASELTINE LAKE & CO. Hazlitt House 28**
**Southampton Buildings Chancery Lane**
**London WC2A 1AT(GB)**

(54) Prosthetic components.

(57) There are described prosthetic components especially : a head (1) which generally takes the form of a segment of a sphere, there being provided an elongate recess (5) for receiving the proximal end of the neck of a prosthetic shaft, the centre line (6) of the recess (5) not passing through the centre of the sphere, to provided eccentricity; a head generally in the form of a segment of a sphere (12), the head being provided with a neck 14 having a recess (17) for receiving the proximal end of a prosthetic shaft, the centre line of the recess (17) not passing through the centre of the sphere, so as to provide eccentricity of the head with respect to the shaft; a head (31) and, integral therewith, an elongate neck (34 ), the head being generally in the form of a segmemt of a sphere and the centre line (38) of the neck where it joins the head not passing through the centre of the sphere to provide eccentricity. There prosthetic heads reduce the likelihood of dislocation when fitted correctly. There is also described a positioning head which enables the correct positioning of the eccentric heads.

EP 0 363 019 A2

## PROSTHETIC COMPONENTS

This invention relates to prosthetic components and, more particularly, to joints which are known as ball and socket joints.

In a human there are two main ball and socket joints, namely the hip joint where a femoral component articulates with respect to an acetabular component; and the shoulder joint where the humeral component articulates with respect to the glenoid component.

The present invention can be applied to ball and socket joints not only in human beings but also in animals.

Although in human joint replacement, hip and shoulder prostheses can be undertaken, for a variety of reasons hip prosthesis is much more common and, to assist in explaining the invention, reference will usually be made in the following description to hip prosthesis and to the components associated with hip prosthesis, but it is to be understood that the invention is not limited to such prosthesis.

The design of prosthetic components for hips falls into two main groups: the design in the first group includes only two parts, namely a femoral component consisting of a shaft and a head, which is fabricated in one piece and may be of metal; and an acetabular socket which may be of plastic. The shaft of the femoral component is inserted into the upper part of the hollow femoral bone, and the plastic socket is mounted in the joint socket of the pelvis.

The design in the second group is modular in that it is based on more than two parts, i.e. the shaft and head of the femoral component are separate pieces, and in many cases the socket comprises two components, namely an outer metal shell and a plastic liner in which the head of the femoral component can articulate.

Both the first and the second groups of design allow cemented or cementless fixation to the bone. In the cemented arrangement the cement provides the necessary fixation, whereas the cementless variety relies on the growth of bone into the porous surface of the prosthetic component or some other form of mechanical fixation.

Often in practice the optimal orientation of the prosthetic components is somewhat difficult to achieve. This is particularly so with the cementless arrangements, in which a good contact between the bone and the prosthetic components is required - a so-called press fit; even if considerable skill and care are exercised, the femoral component may tend to orientate itself according to the shape of the inner canal of the femur, rather than according to the intentions of the surgeon.

One unfortunate consequence of an unsatisfactory relative positioning of the components is that the head may dislocate out of the socket, which is very painful for the patient and which in most cases necessitates anaesthesia during reduction, i.e. restoration of the dislocated joint to its normal position.

Another consequence of an unsatisfactory relative position of the prosthetic components is that it may lead to more frequent or even continuous impingement of the neck (i.e. that part between the shaft and the head) of the femoral component on the rim of the socket, which may in turn lead, in due course, to loosening of the components.

Unsatisfactory positioning of the prosthetic components may result in dislocation at any time after the operation, owing to the neck acting as a lever arm when impinging on the rim of the acetabular socket, thus lifting the head out of the socket. The risk of this can only be avoided by positioning the components within certain narrow limits, within which the interface between the components is optimal. However, as mentioned, the optimal position is not always achieved.

Some non-modular systems have been produced in which the head is offset with respect to the neck of the shaft. Although such eccentricity decreases the risk of dislocation when the shaft is correctly positioned, such systems are not able to aid the surgeon to position the component correctly as once the shaft is inserted into the femur it is not possible to correct the position of the head, such that incorrect positioning is still a problem with such systems.

With the modular prosthetic systems mentioned above, a certain degree of correction of poor positioning is possible, as the surgeon may be given the choice of different liners for the socket and different heads to locate in the socket, but conventionally available modular systems do not totally overcome the problems of incorrect positioning. Sockets with part of the rim built up to prevent dislocation have been manufactured by several manufacturers but this cannot prevent impingement, and thus it seems likely that although the risk of dislocation may be reduced or avoided in the short term, the long term effect of constant leverage attributable to impingement probably contributes to aseptic loosening of the components, which currently is a major problem in total joint replacement.

With a view to reducing the incidence of unsatisfactory positioning of the prosthetic components where artificial ball joints are used, eccentric sockets have been used, but generally they have not all proved to be altogether successful.

Furthermore, assuming that the shift in the direction of pull of the main muscles associated with incorrect positioning of prosthetic components is of importance with regard to dislocation, this is not remedied with sockets having built-up walls in which the direction of pull remains the same.

According to a first aspect of the present invention, there is provided a prosthetic head substantially in the form of a segment of a sphere being provided in the planar surface of the segment with a recess capable of receiving a neck of a prosthetic shaft, wherein the recess is eccentric to the head as the centre line of the recess does not pass through the centre of the sphere.

According to a second aspect of the present invention, there is provided a prosthetic head substantially in the form of a segment of a sphere, being provided on the planar surface of the segment with a neck having a recess capable of receiving a portion of a prosthetic shaft, wherein the neck or the recess is eccentric to the head as the centre line of the neck or the recess does not pass through the centre of the sphere.

According to a third aspect of the present invention, there is provided a prosthetic head substantially in the form of a segment of a sphere being provided on the planar surface with an elongate neck capable of being accommodated in a recess provided in a prosthetic shaft, wherein the neck is eccentric to the head as the centre line of the neck where it joins the planar surface of the head does not pass through the centre of the sphere.

A head according to the first, second and third aspects of the invention allows correct positioning to take place, even after insertion of the shaft into the femur, as the head is independent of the shaft, allowing the surgeon to move the head relative to the shaft until correct positioning is achieved. Further the eccentricity of the head decreases the risk of dislocation when the joint is incorrectly positioned and also increases the range of motion possible by a patient in which a prosthetic joint using a head according to the first, second or third aspects of the invention has been inserted.

For heads according to the first, second or third aspect of the invention, the centre line of the recess or of the neck may be inclined to the planar surface of the head at an angle other than 90°. Preferred angles are in the range of 5° to 20° on either side of 90°.

A head according to the third aspect of the present invention may be provided with an elongate neck which may be curved in the region adjacent to the head so that the centre line of the neck where it joins the planar surface is inclined to the centre line of the neck at its end to be inserted into the shaft.

The centre line through the neck or recess may lie on a radius from the centre of the sphere, and/or it may be parallel to that radius.

The neck provided in the second or third aspects of the present invention is preferably integral with the head.

For the above aspects of the invention the outer diameter of the head is typically in the range of from 22 to 60 mm, thus being suited for total joint replacements in which the head articulates with an artificial socket, and for hemiarthroplasty in which only the femoral component, which articulates with respect to the human joint socket, is replaced.

The centre line of the neck or the recess may be offset from 0 to 5 mm with respect to the central point of the planar surface of the head. It is envisaged that heads of varying offsets should be available to a surgeon so that the correct offset may be selected.

Rotational stability between the shaft and the head may be achieved by a means of a tapered interface between the head and neck or neck and shaft as appropriate, or by a spline or other mechanical means.

Heads of various degrees of eccentricity are envisaged, which would allow the surgeon to match the eccentric head to the patients needs. The degree of eccentricity of the head may be stated on the head itself, e.g. on the planar surface, and may be indexed in degrees, with zero indicating the head in valgus ie. where the foot is turned outwards to the maximum degree possible in which position forward/backward rotation is not possible, and 180 indicating the head in varus i.e. where the foot is turned inwards to the maximum degree possible in which position forward/backward rotation is not possible. Positions in which the head is pointing forward relative to the neck or recess centre line may be denoted by positive indexing, while negative indexing may denote the opposite.

To enable there to be an increase in the tension in the muscles around the hip joint the neck of a head according to the second aspect of the present invention may be elongated so that the recess is not sunk so far into the head, but is provided within the elongated neck.

According to a fourth aspect of the present invention there is provided a positioning head substantially in the form of a segment of a sphere being provided with indexing on its exterior surface from which the relative position of the positioning head relative to a socket may be determined.

The positioning head may be provided with a shell in which the head may be located, the shell being intended for location in a socket having a planar opening, and having means allowing insertion in a certain position only and allowing rotation about an axis perpendicular to the planar opening of the socket but preventing rotation about other axes.

The indexing on the positioning head may take any convenient form but a preferred form constitutes a plurality of parallel lines, like the lines of latitude on a globe, the lines lying in their respective planes which are parallel to the planar face of the head.

The positioning head is not an eccentric head and is provided with a non-eccentric recess to receive the neck of a shaft or is provided with a non-eccentric neck which can locate with the shaft.

A positioning head according to the fourth aspect of the present invention is advantageous as enables a surgeon to determine the correct relative positioning of a prosthetic head in a socket. The surgeon reads the value indexed on the correctly positioned positioning head and then refers to a table, from which he can determine which particular eccentric head according to the first to third aspects of the present invention is to be employed in the case of a particular patient.

There should also be correct alignment between the head and the shaft.

If the shaft is to be inserted after alignment with the head the positioning head may be provided with preferably a cooperating tongue and groove (to prevent rotation).

Alternatively the trial head may be provided with a secondary index such as four perpendicular lines, equivalent to lines of longitude, perpendicular to the preferred index of a plurality of parallel lines, equivalent to lines of latitude.

By reading both indexes (provided by the intersections between the lines of latitude and the lines of longitude) which are only just visible as two sets of perpendicular surface lines, one quadrant apart, when a patient is lying supine and when the leg and foot are placed 180° extended and pointing straight forward respectively, and by referring the indexed values to a table, a surgeon can ascertain not only the optimal eccentric head to be used but also the optimal degree of rotation to be used between the head and the shaft.

The positioning head may be provided with the shell to increase the reliability of the readings. The trial head is smaller if a shell is provided. The shell can be generally hemispherical and fit inside the acetabular socket, and a lip on the circumference of the shell can be located in a chamfer at the edge of the socket, to secure the shell in position and to prevent it rotating even when the positioning head is rotated. Thus, in practice, the surgeon will align the positioning head with the shell parallel to the acetabular rim and the positioning head in the position where the leg is 180° extended and the foot is pointing straight ahead, read the indexes of the positioning head, consult the table, choose the right prosthetic head, and insert it in the right position.

For a better understanding of the present invention and to show how the same may be carried into effect, reference will now be made, by way of example, to the accompanying drawings, in which:

Figure 1 is a section through an eccentric head in accordance with the first aspect of the present invention;

Figure 2 is a section through one embodiment of a head in accordance with the second aspect of the present invention and a shaft which has been inserted into the head;

Figure 3 is a section through a different embodiment of a head in accordance with the second aspect of the present invention and a shaft which has been inserted into the head;

Figure 4 is a section through a head in accordance with the third aspect of the present invention and a shaft into which the head has been inserted; and

Figure 5 is a section through a positioning head in accordance with the fourth aspect of the present invention, and a socket in which it is located.

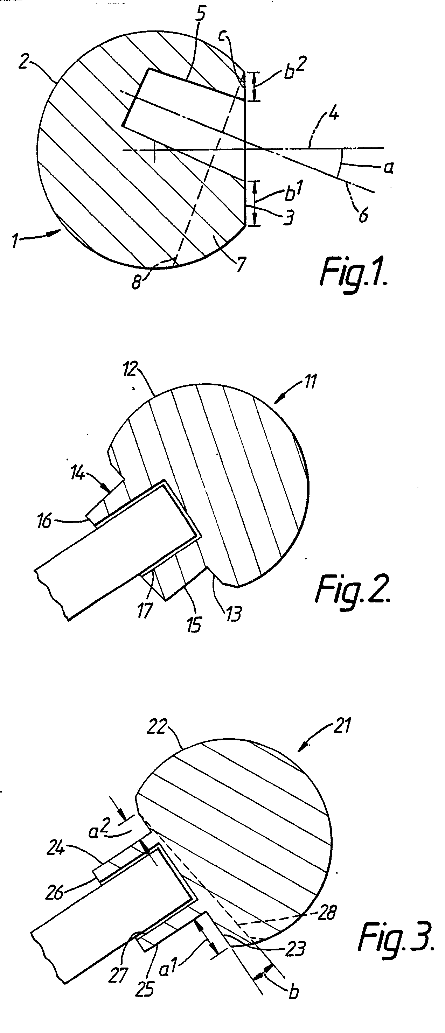

Referring firstly to Figure 1, there is shown a head (1) in the form of a segment of a sphere having a partially spherical external surface (2) and a planar surface (3). The line (4) shown in Figure 1 passes through the centre of the sphere and through the centre of the planar face (3).

Provided in the planar face (3) is a recess (5) for accommodating the neck (not shown) of a femoral shaft (also not shown). The recess (5) has a centre line (6).

The centre line (6) of the recess (5) does not pass through the centre of the planar face (3); instead, the centre line (6) is slightly offset as indicated by the fact that the dimension $b^1$ exceeds the dimension $b^2$. In addition the centre line (6) intersects the line (4) at an angle a. The angle between the centre line (6) of the recess and the line (4) can be, for instance, in the range from 5 to 20°.

It would, theoretically, be possible to remove that part of the head marked (7) which lies to the right of the discontinuous line (8) but it is preferred for it to remain as then there is a greater portion of the head within the socket which reduces the risk of dislocation.

Referring now to Figure 2, there is shown here a head (11) which has an integral neck (14). The head is again in the form of a segment of a sphere and hence has a partially spherical external surface (12), and a planar surface (13). Projecting normally, i.e. at 90°, and centrally from the planar surface (13) is the neck (14). The neck (14) has a cylindrical side wall (15) and a planar end wall (16). The neck (14) is provided with a recess (17). The centre line of the recess (17) is inclined with respect to the centre line of the neck (14), such that the recess is mounted off-centre with respect to the centre of the end face (16) of the neck (14), and the centre line through the recess (17) does not pass through the centre of the head (11).

The arrangement shown in Figure 3 is a variation on that shown in Figure 2. The head is again in the form of a segment of a sphere and has a partially spherical surface (22) and a planar surface (28), but there is a tapered extension (23) mounted on the planar surface (28). The neck (24) is mounted on the extension (23) and hence is angled to the planar surface (28) of the head (11). The neck (24) is mounted normally, i.e., at 90°, with respect to the extension (23) but offset with respect to its centre. In the arrangement of Figure 3 the neck (24) has a cylindrical external side wall (25) and an end face (26), and is provided with a recess (27), the centre line of the recess (27) is parallel to the centre line of the neck (24} and lies on that centre line. The eccentricity is achieved by the provision of the extension (23) as the neck (24) is offset with respect to that extension (23). The degree of offset of the neck (24) with regard to the extension (23) can be seen by the fact that the dimension $a^1$ exceeds the dimension $a^2$. The extra material of the extension (23) can be quantified by a measurement of the angle b.

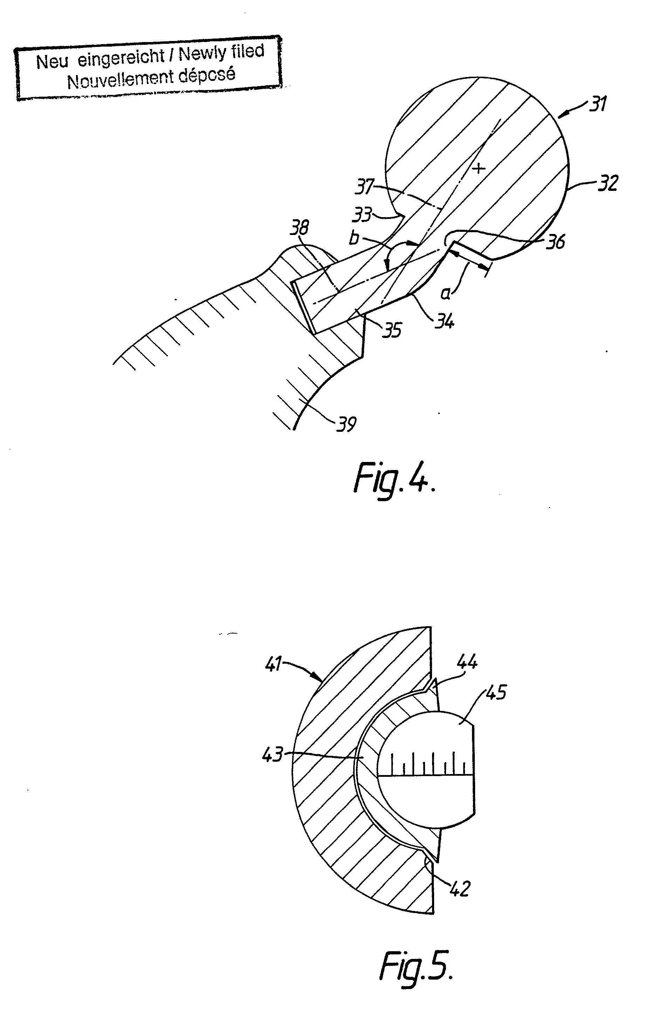

A different arrangement is shown in Figure 4 where the neck, instead of being integral with the shaft and inserted in the recess in the head, is integral with the head and is secured in a recess in the shaft. The Figure 4 arrangement shows a head (31) in the form of a segment of a sphere, having a partially spherical external surface (32) and a planar surface (33). Integral with the head (31) is a neck (34) which is elongate and rectilinear over a distal part (35) of its length but is curvilinear over a proximal part (36) of its length. Where the neck (34) joins the planar surface (33) of the head (31) the neck (34) is perpendicular to the planar surface (33); however the centre line (37) through the neck (34) is offset with respect to the centre of the planar surface (33), so that the centre line (37) of the neck does not pass through the centre of the sphere. Further the centre line (37) of the neck where it joins the planar surface (33) is inclined with respect to the centre line (38) of the neck in its rectilinear part (35). The distal end region (35) of the neck (34) is located in a suitable recess in the shaft (39) of the femoral component.

Figure 5 shows a different aspect of the invention, in fact a positioning head within its own shell, which, in turn, is accommodated within an artificial socket.

The socket (41) is general hemispherical and has a slightly chamfered internal rim (42). The shell (43) is also generally hemispherical in internal and external appearance but is provided on its external face at its rim with a lip (44) intended to cooperate with the chamfered region (42) of the socket (41). The arrangement of chamfered region (42) and lip (44) prevents the shell from rotating in such a way that the rim of the shell disappears within the socket. The shell (43) and socket (41) are dimensioned such that there is a good but loose fit so that the shell (43) can be freely removed from the socket (41). Located snugly within the shell (43) is the positioning head (45). The shell (43) fits the positioning head (45) sufficiently firmly so that, when the positioning head (45) is removed, the shell (43) is removed with the head and remains in the same position relative to the head (45) so that index numbers on the positioning head can clearly be read by a surgeon.

The positioning head is more than hemispherical, and is provided with index numbers which run from its truncated region towards its pole. As mentioned earlier the indexes could be in the form of parallel lines, like lines of latitude around the globe, with four longitude lines also present. However, the lines of latitude could be discontinuous and need only be present in the region where they intersect with the four lines of longitude which are perpendicular.

The positioning head is preferably provided with a recess (for accommodating the neck of a shaft), the centre line of the recess being normal to the plane of truncation of the spherical positioning head (45) and passing through the centre of the sphere of the head (45). The positioning head has, as a means of aligning it with a suitable alignment mark on the neck of the shaft, one of the longitudinal lines.

With the shaft suitably located in the femur of a patient and with a conventional (non-eccentric) neck of the shaft entering the recess of the positioning head and with the positioning head (45) and its shell (43) located in the socket (41), the surgeon can extend the leg to 180° and point ahead the foot of a patient lying on the operating table. By extending the leg and pointing ahead the foot, the resulting movement of the femur will cause the shaft and neck to cause rotation of the positioning head (45) within the shell (43). The positioning head and its associated shell can then be removed from the socket (41) and the surgeon can read the index markings on adjacent lines of longitude. He can then consult the appropriate table to determine the degree of eccentricity needed and the degree of rotation to be used in its positioning. By suitably selecting the prosthetic head and by appropriately fitting at the right angle, the surgeon can greatly reduce the likelihood of any impingement and dislocation and increase the range of motion possible with the resulting joint.

The surgeon can have access to a range of eccentric heads, with angles in the range from, for instance, 0 to 25°; convenient eccentric heads could be angled to 5, 7.5, 10, 12.5 or 15°.

**Claims**

1. A prosthetic head substantially in the form of a segment of a sphere, being provided in the planar surface of the segment with a recess capable of receiving a neck of a prosthetic shaft, wherein the recess is eccentric to the head as the centre line of the recess does not pass through the centre of the sphere.

2. A prosthetic head substantially in the form of a segment of a sphere, being provided on the planar surface of the sphere with a neck having a recess capable of receiving a portion of a prosthetic shaft, wherein the neck or recess is eccentric to the head as the centre line of the neck or the recess does not pass through the centre of the sphere.

3. A prosthetic head substantially in the form of a segment of a sphere, being provided on the planar surface with an elongate neck capable of being accommodated in a recess provided in a prosthetic shaft, wherein the neck is eccentric to the head as the centre line of the neck where it joins the planar surface of the head does not pass through the centre of the sphere.

4. A prosthetic head according to claim 1, 2 or 3, wherein the centre line through the recess or the neck is inclined to the planar surface at an angle other than at 90°.

6. A prosthetic head according to any preceding claim, wherein the centre line through the recess or neck is on, or parallel to, a radius from the centre of the sphere.

7. A prosthetic head according to any preceding claim, wherein the centre line of the neck or the recess is offset from 0 to 5 mm with respect to the central point of the planar surface.

8. A positioning head substantially in the form of a segment of a sphere, being provided with indexing on its exterior surface from which the relative position of the positioning head relative to a socket may be determined.

9. A positioning head according to claim 8, further provided with a shell in which the positioning head may be located, the shell being intended for location in a socket having a planar opening, and having means allowing insertion in a certain position only in the socket and for allowing rotation about an axis perpendicular to the planar opening of the socket but preventing rotation about other axes.

10. A positioning head according to claim 8 or 9, wherein the indexing comprises a plurality of parallel lines, like the lines of latitude on a globe, the lines lying in their respective planes which are parallel to the planar surface of the head.

11. A positioning head according to claim 10, provided with secondary indexing in the form of four perpendicular lines, equivalent to lines of longitude, perpendicular to the plurality of parallel lines, equivalent to lines of latitude.

12. A positioning head according to claim 9, 10 or 11, wherein the means for preventing rotation about the other axes is a lip on the circumference of the shell, which can be located in a chamfer at the edge of the socket.

*Fig.1.*

*Fig.2.*

*Fig.3.*

Fig.4.

Fig.5.